# EUROPEAN PATENT SPECIFICATION

(11) **EP 1 693 374 B1**
(45) Date of publication and mention of the grant of the patent: **15.10.2008**
(21) Application number: 04807132.8
(22) Date of filing: 09.12.2004
(51) Int. Cl.: C07D 495/04, A61K 31/55, A61P 13/00

(54) **MICROCRYSTAL AND MEDICINAL PREPARATION CONTAINING THE SAME**
MIKROKRISTALL UND MEDIZINISCHE ZUSAMMENSETZUNG, DIE DIESEN ENTHÄLT
PREPARATION MICROCRISTALLINE ET PREPARATION MEDICINALE CONTENANT CETTE DERNIERE

(30) Priority: 11.12.2003 JP 2003413725
(43) Date of publication of application: 23.08.2006
(73) Proprietor: KYOWA HAKKO KOGYO CO., LTD., Chiyoda-ku, Tokyo 100-8185 (JP)
(72) Inventor: IZAWA, Naoto, Princeton, NJ 08540 (US); SATOH, Norie 470-1-304, Shimotogari, Shizouka 411-0943 (JP); YAGI, Nobuhiro c/o Pharmaceutical Research Centre,, Nagaizumi-cho,Sunto-gun,Shizouka 411-8731 (JP); OUCHI, Kazue c/o Pharmaceutical Research Center, Nagaizumi-cho, Sunto-gun, Shizuoka 411-8731 (JP); NARITA, Shoichi c/o Fuji Plant, Nagaizumi-cho,Sunto-gun,Shizuoka 411-8731 (JP); AOKI, Noboru c/o Pharmaceutical Research Center, Nagaizumi-cho, Sunto-gun, Shizuoka 411-8731 (JP)
(74) Representative: Vossius & Partner
(86) International application number: PCT/JP2004/018773
(87) International publication number: WO 2005/056561

(56) References cited:
- WO-A1-00/00492
- WO-A1-02/13823
- WO-A1-98/46587
- WO-A1-02/078710
- WO-A1-02/078711
- WO-A1-02/078712
- WO-A1-03/041704
- WO-A2-02/45691
- JP-A- 2002 053 580

## Description

### Technical Field

The present invention relates to crystals of (S)-(+)-3,3,3-trifluoro-2-hydroxy-2-methyl-N-(5,5,10-trioxo-4,10-dihydrothieno[3,2-c][1]benzothiepin-9-yl) propaneamide [hereinafter referred to as Compound (I)] and a pharmaceutical formulation comprising the crystals.

### Background Art

Compound (I) is useful for the treatment of various diseases and symptoms including urinary frequency, urinary incontinence, overactive bladder and the like. Its crystals and synthesis method have been known, and examples of the dosage form thereof have been also known (refer to, for example, WO 98/46587, WO 02/78710, WO 02/78711, WO 02/78712 / WO 03/041704 and Japanese Published Unexamined Patent Application No. 53580/2002). Crystals of Compound (I) obtained by the methods described in the foregoing cited documents are plate crystals having an average particle size of from 100 to several hundred µm, and are low in solubility in water and absorbability in oral administration. The crystals of Compound (I) are not stable in properties such as dissolution property, absorbability and stability in steps for preparing pharmaceutical formulations. Although the improvement in absorbability is observed by forming a solution including this compound, there is still the problem that the stability is low. WO 00 00492 deals with the preparation of an oral solid formulation of L-aminoadipic acid, WO 02 13823 is concerned with formulations of torsemide having a mean particle diameter of less than 200 microns, WO 02 45691 deals with laboratory scale milling processes relating to a series of drugs which does not include Compound (1).

### Disclosure of the Invention

An object of the present invention is to provide crystals of Compound (I) having good solubility, good absorbability, good stability and a pharmaceutical formulation comprising the crystals.

The invention relates to the following (1) to (9).
(1) A microcrystal of (S)-(+)-3,3,3-trifluoro-2-hydroxy -2-methyl-N-(5,5,10-trioxo-4,10-dihydrothieno[3,2-c][1] benzothiepin-9-yl)propaneamide represented by the following formula 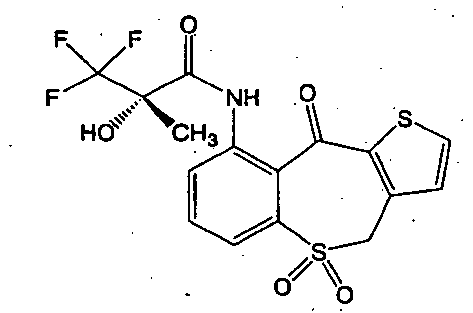 which has an average particle size of 80 µm or less.
(2) The microcrystal according to the above (1), wherein the average particle size of the microcrystal of (S)-(+)-3,3,3-trifluoro-2-hydroxy-2-methyl-N-(5,5,10-trioxo-4,10-dihydrothieno[3,2-c][1]benzothiepin-9-yl) propaneamide is from 1 to 40 µm.
(3) The microcrystal of (S)-(+)-3,3,3-trifluoro-2-hydroxy-2-methyl-N-(5,5,10-trioxo-4,10-dihydrothieno[3,2-c ][1]benzothiepin-9-yl)propaneamide according to the above (1) or (2), wherein a crystallinity thereof is 15% or more.
(4) The microcrystal of (S)-(+)-3,3,3-trifluoro-2-hydroxy-2-methyl-N-(5,5,10-trioxo-4,10-dihydrothieno[3,2-c ] [1]benzothiepin-9-yl)propaneamide according to the above (1) or (2), wherein the crystallinity thereof is 25% or more.
(5) The microcrystal of (S)-(+)-3,3,3-trifluoro-2-hydroxy-2-methyl-N-(5,5,10-trioxo-4,10-dihydrothieno[3,2-c ][1]benzothiepin-9-yl)propaneamide according to the above (1) or (2), wherein the crystallinity thereof is 45% or more.
(6) The microcrystal of (S)-(+)-3,3,3-trifluoro-2-hydroxy-2-methyl-N-(5,5,10-trioxo-4,10-dihydrothieno[3,2-c ][1]benzothiepin-9-yl)propaneamide according to the above (1) or (2), wherein the crystallinity thereof is 75% or more.
(7) A pharmaceutical formulation comprising the microcrystals described in any one of the above (1) to (6).
(8) A process for producing the microcrystals described in any one of the above (1) to (6), comprising a step of dry-pulverizing (S)-(+)-3,3,3-trifluoro-2-hydroxy-2-methyl -N-(5,5,10-trioxo-4,10-dihydrothieno[3,2-c][1]benzothiepin -9-yl)propaneamide which has an average particle size of 80 µm or more.
(9) The process for producing the microcrystals according to the above (8), wherein a jet mill is used in the pulverization step.

In the present specification, the term "(S)-(+)-3,3,3-trifluoro-2-hydroxy-2-methyl-N-(5,5,10-trioxo-4,10-dihydrothieno[3,2-c][1]benzothiepin-9-yl) propaneamide" ("Compound (I)") means amorphous Compound (I), crystalline Compound (I) or a mixture of them.

The expression "(S)-(+)-3,3,3-trifluoro-2-hydroxy-2-methyl-N-(5,5,10-trioxo-4,10-dihydrothieno[3,2-c][1]benzothiepin-9-yl)propaneamide having an average particle size of 80 µm or less" ("microcrystal(s) of Compound (I)") is referred to crystalline Compound (I) having an average particle size of 80 µm or less. Among them, microcrystal(s) having an average particle size of from 1 to 40 µm is/are preferable. The "microcrystal(s) of Compound (I)" having a crystallinity of 15% or more is/are more preferable. Among them, the "microcrystal(s) of Compound (I)" having a crystallinity of 25% or more is/are further preferable. The "microcrystal(s) of Compound (I)" having a crystallinity of 45% or more is /are still more preferable. The "microcrystal(s) of Compound (I)" having a crystallinity of 75% or more is/are most preferable.

"Microcrystal(s) of Compound (I)" of the present invention, can be prepared by pulverization and/or sieving of "crystal(s) of (S)-(+)-3,3,3-trifluoro-2-hydroxy -2-methyl-N-(5,5,10-trioxo-4,10-dihydrothieno[3,2-c][1] benzothiepin-9-yl)propaneamide having an average particle size of 80 µm or more" ("crystals of Compound (I)") which is/are obtained by the method described in, for example, WO 98/46587, Japanese Published Unexamined Patent Application No. 53580/2002 or the like, or by the method similar thereto. The pulverization and the sieving may be properly conducted in combination several times. The pulverization can be conducted by a pulverizer generally used, and it is especially preferable to conduct dry-pulverization. The dry-pulverization means a method in which a solid is pulverized in air or the like. As the pulverizer, for example, a mortar, Mechanomill and a jet mill are used. The "microcrystal(s) of Compound (I)" having a desired average particle size or a desired average particle size and crystallinity can be obtained by appropriately controlling, for example, a rotational speed of the pulverizer, a feed rate of the "crystal(s) of Compound (I)" and a time required for pulverization. Among them, the pulverization by the jet mill is preferable. For example, the "crystal(s) of Compound (I)" can be pulverized under pulverization pressure of from 0.01 to 1.0 MPa by feeding the "crystal(s) of Compound (I)" at a rate of from 0.1 to 1,000 g/min.

In regard to the pharmaceutical formulation comprising the "microcrystals of Compound (I)" of the present invention, any pharmaceutical formulation may be used so long as it comprises the "microcrystals of Compound (I) " described above. Examples thereof include (a) a formulation obtained by mixing the "microcrystal(s) of Compound (I)" formed by the methods described above with additives and preparing a formulation, (b) a formulation obtained by mixing the "microcrystal(s) of Compound (I)" formed by the methods described above with additives, pulverizing and/or sieving the resulting mixture in a similar manner to the method for preparing the "microcrystal(s) of Compound (I)" described above and then preparing a formulation, (c) a formulation obtained by forming a solid dispersion from "Compound (I)" and a dispersant, then mixing the solid dispersion with additives and preparing a formulation, and the like. Incidentally, the content of the "microcrystal(s) of Compound (I)" in the pharmaceutical preparation of the present invention is preferably from 0.001 to 80%, more preferably from 0.1 to 50%.

The solid dispersion is a solid dispersion prepared from "Compound (I)" or the "crystals of Compound (I)" and a dispersant which can disperse the above. The average particle size, or the average particle size and the crystallinity of the "microcrystals of Compound (I)" as described above. In regard to the dispersant, for example, polymer substances such as hydroxypropyl methylcellulose (HPMC), polyvinylpyrrolidone (PVP), hydroxypropyl cellulose (HPC) and the like are preferable. In addition, "Compound (I)" or the "crystals of Compound (I)" and the dispersant are combined in a combination ratio of preferably 1:0.1 to 1:10 (ratio by weight), more preferably 1:0.1 to 1:4 (ratio by weight). The solid dispersions of the present invention, can be prepared by general methods such as a mixing/pulverizing method and a solvent method from "Compound (I)" or "crystals of Compound (I)", which is/are obtained by the method described, for example, in WO98/46587, Japanese Published Unexamined Patent Application No. 53580/2002, or the method similar thereto, and the dispersant.

Examples of the mixing/pulverizing method include a method which consists of mixing the "crystals of Compound (I)" and the dispersant in a blender or the like, and pulverizing by a generally used pulverizer such as a mortar, Mechanomill or a jet mill; or the like. For example, the rotational speed of the pulverizer, the feed rate of the "crystals of Compound (I)", time required for pulverization and the like, are appropriately controlled to obtain the solid dispersion comprising the "microcrystals of Compound (I)" having a desired average particle size, or a desired average particle size and a desired crystallinity. Among them, the pulverization by the jet mill is preferable.

Examples of the solvent method is a method which consists of dissolving or dispersing "Compound (I)" or the "crystals of Compound (I)" in an organic solvent with a dispersant and then removing the organic solvent by a general method under reduced pressure or ordinary pressure, and the like. Specifically, for example, a fluidized bed granulator, a stirring granulator, a spray granulator, a spray-drying granulator, a vacuum drying granulator and the like can be used and, if desired, pulverizing method using a generally used pulverizer such as a mortar, Mechanomill or a jet mill is used may be combined therewith. Examples of organic solvents suitable for dissolving "Compound (I)" or "the crystals of Compound (I)" include halogenated hydrocarbon such as dichloromethane, dichloroethane and chloroform; ketone such as acetone and methyl ethyl ketone; alcohol such as methanol and ethanol; ether such as tetrahydrofuran; esters such as ethyl acetate; and amide such as dimethylformamide and dimethylacetamide.

Examples of the additive include an excipient, a binder, a disintegrator, a lubricant, a plasticizer, a surfactant, a coating agent, a colorant, a corrigent and an acidifying agent, and they may be appropriately used depending upon the type of the preparation.

Examples of the excipient include a sugar such as sucrose, mannitol and lactose; starch such as corn starch and potato starch; and cellulose such as crystalline cellulose

Examples of the binder include polyvinyl alcohol, hydroxypropylcellulose, gelatin, methylcellulose, ethylcellulose and polyvinylpyrrolidone.

Examples of the disintegrator include starch such as corn starch and potato starch; crystalline cellulose; and crospovidone.

Examples of the lubricant include magnesium stearate and talc.

Examples of the plasticizer include plant oil and glycerin.

Examples of the surfactant include sodium lauryl sulfate, polysorbate 80 and fatty acid esters.

Examples of the coating agent include sucrose, hydroxypropylcellulose and the like.

Examples of the colorant include food dyes, tar dyes and the like. Examples of the corrigent include sodium saccharide, aspartame and stevia. Examples of the acidifying agent include citric acid, malic acid, tartaric acid and the like.

The expression "crystal(s) of (S)-(+)-3,3,3-trifluoro-2-hydroxy-2-methyl-N-(5,5,10-trioxo-4,10-dihydrothieno[3,2-c][1]benzothiepin-9-yl) propaneamide where crystallinity is 15% or more" (that is "crystal(s) of Compound (I) where crystallinity is 15% or more") means crystalline Compound (I) having a crystallinity of 15% or more. Among them, the crystalline Compound (I) having a crystallinity of 25% or more is preferable, and the crystalline Compound (I) having a crystallinity of 45% or more is more preferable, and the crystalline Compound (I) having a crystallinity of 75% or more is most preferable.

The compound of the present invention can be obtained by the method described in WO98/46587, Japanese Published Unexamined Patent Application No. 53580/2002.

In regard to the pharmaceutical formulation comprising the "crystals of Compound (I) having a crystallinity of 15% or more", any formulations may be used so far as it is a pharmaceutical formulation comprising the "crystals of Compound (I) having a crystallinity of 15% or more" described above. Examples of the process for producing the pharmaceutical formulations include the process similar to the above-described processes for producing the pharmaceutical formulations comprising the "microcrystals of Compound (I)".

The solid dispersion comprising the "crystals of Compound (I) having a crystallinity of 15% or more" and a dispersant is a solid dispersion which is prepared from "Compound (I)" or the "crystals of Compound (I)", and a dispersant which can disperse it. The crystalline parts of Compound (I) in the solid dispersion have a crystallinity of 15% or more. Examples of the process for producing the solid dispersions include a process similar to the above-described process for producing solid dispersions comprising the "microcrystals of Compound (I)" and a dispersant. With regard to the dispersant, for example, HPMC, PVP, HPC and the like are preferable. In addition, "Compound (I)" or the "crystals of Compound (I)" and the dispersant are combined in a combination ratio of preferably 1:0.1 to 1:10 (ratio by weight), more preferably 1:0.1 to 1:4 (ratio by weight).

Examples of the dosage form of the pharmaceutical formulation of the present invention include tablets such as sugar-coated tablets, diluted powders, granules, capsules, pills, troches and suspension liquids for oral application, and the formulations can be manufactured by combining the steps for preparing formulations, which have been well known in the technical field of pharmaceutics, such as a mixing step, a pulverizing step, a sieving step, a granulation step, a milling step, a tabletting step, a drying step, a capsule-filling step and a coating step.

The present invention is illustrated more specifically below by referring to Examples, Comparative Examples and Test Examples.

### Best Mode for Carrying Out the Invention

### Example 1

Unpulverized crystals of Compound (I) obtained by the method described in Japanese Published Unexamined Patent Application No. 53580/2002 were pulverized under pulverization pressure of 0.4 MPa using a jet mill (A-0 Jet/Seishin Enterprises) while feeding at a rate of 1 g/min. An average particle size of Compound (I) before and after the pulverization was measured using a laser beam scattering-type particle size distribution analyzer (LDSA-1300A/Tonichi Computer Applications). Further, a crystallinity of Compound (I) before and after the pulverization was measured using a powder X-ray diffractometer (PW 3050/PANalytical). The results are shown in Table 1.

**Table 1**

| Before pulverization | | After pulverization | |
|---|---|---|---|
| Average particle size | Crystallinity | Average particle size | Crystallinity |
| 90 µm | 90% | 5 µm | 80% |

### Example 2

Unpulverized crystals of Compound (I) obtained by the method described in Japanese Published Unexamined Patent Application No. 53580/2002 were cooled with ice, and then pulverized under pulverization pressure of 0.6 MPa using a jet mill while feeding at a rate of 2 kg/hr. An average particle size of Compound (I) before and after the pulverization was measured using a laser beam scattering-type particle size distribution analyzer (Mastersizer 2000/Malvern Instruments). Further, a crystallinity of Compound (I) before and after the pulverization was measured using a powder X-ray diffractometer (PW 3050/PANalytical). The results are shown in Table 2.

**Table 2**

| Before pulverization | | After pulverization | |
|---|---|---|---|
| Average particle size | Crystallinity | Average particle size | Crystallinity |
| 90 µm | 90% | 10 µm | 80% |

### Example 3

0.8 g of Compound (I) obtained by the pulverization with the jet mill in Example 2 and 3.2 g of hydroxypropylmethyl cellulose were mixed in a plastic bag to form a mixture of Compound (I) and hydroxypropylmethyl cellulose.

### Example 4

7 g of unpulverized Compound (I) obtained by the method described in Japanese Published Unexamined Patent Application No. 53580/2002 were pulverized for 1 hour, 4 hours and 24 hours respectively using an automatic mortar (ANM 1000 model/Nitto). Further, an average particle size of Compound (I) before and after the pulverization was measured using a laser beam scattering-type particle size distribution analyzer (LDSA-1300A/Tonichi Computer Applications). The results are shown in Table 3.

**Table 3**

| Pulverization time | Before pulverization | 1 hour | 4 hours | 24 hours |
|---|---|---|---|---|
| Average particle size (µm) | 90 | 11 | 4 | 4 |

### Example 5

4 g of unpulverized Compound (I) obtained by the method described in Japanese Published Unexamined Patent Application No. 53580/2002 were pulverized for 1 hour and 4 hours respectively using an automatic mortar (ANM 1000 model/Nitto). Further, a crystallinity of Compound (I) before and after the pulverization was measured using a powder X-ray diffractometer (PW 3050/PANalytical). The results are shown in Table 4.

**Table 4**

| Pulverization time | Before pulverization | 1 hour | 4 hours |
|---|---|---|---|
| Crystallinity (%) | 90 | 49 | 29 |

### Example 6

0.8 g of Compound (I) obtained by the pulverization with the mortar for 1 hour in Example 5 and 3.2 g of-hydroxypropylmethyl cellulose were mixed in a plastic bag to obtain a mixture of Compound (I) and hydroxypropylmethyl cellulose.

### Example 7

0.8 g of Compound (I) obtained by the pulverization with the mortar for 4 hours in Example 5 and 3.2 g of hydroxypropylmethyl cellulose were mixed in a plastic bag to obtain a mixture of Compound (I) and hydroxypropylmethyl cellulose.

### Example 8

Compound (I) obtained by the pulverization with the jet mill in Example 1 was dispersed in a 0.5% methyl cellulose aqueous solution. The dispersion in an amount corresponding to 25 mg of Compound (I) was filled into a capsule. In this manner, the capsule was obtained.

### Example 9

Compound (I) obtained by the pulverization with the mortar for 24 hours in Example 4 was dispersed in a 0.5% methyl cellulose aqueous solution. The dispersion in an amount corresponding to 25 mg of Compound (I) was filled into a capsule. In this manner, the capsule was obtained.

### Example 10

25 mg of Compound (I) obtained by the pulverization with the jet mill in Example 1 was filled into a capsule. In this manner, the capsule was obtained.

### Example 11

Tablets were obtained in a usual manner using 25 mg of Compound (I) obtained by the pulverization with the jet mill in Example 1, 60 mg of lactose, 30 mg of potato starch, 2 mg of poly(vinyl alcohol), 1 mg of magnesium stearate and a trace amount of a tar dye.

### Comparative Example 1

Unpulverized Compound (I) obtained by the method described in Japanese Published Unexamined Patent Application No. 53580/2002 was dispersed in a 0.5% methyl cellulose aqueous solution, and the dispersion in an amount corresponding to 25 mg of Compound (I) was filled into a capsule. In this manner, the capsule was obtained.

### Comparative Example 2

25 mg of unpulverized Compound (I) obtained by the method described in Japanese Published Unexamined Patent Application No. 53580/2002 was filled into a capsule. In this manner, the capsule was obtained.

### Test Example 1

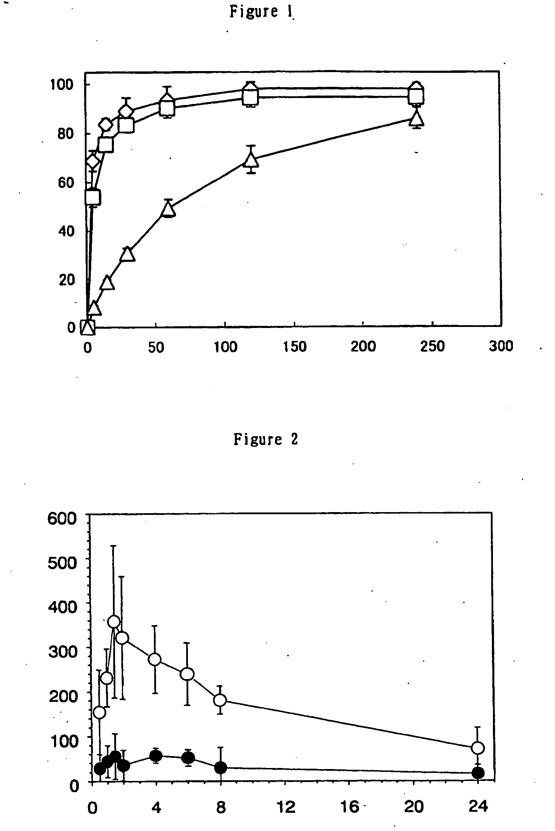

A dissolution test (puddle method) of the capsule obtained in each of Example 8 and 9, and Comparative Example 1 was performed. The results are shown in Fig. 1.

According to Fig. 1, it has been found that the capsule containing Compound (I) pulverized with the jet mill (Example 8) and the capsule containing Compound (I) pulverized with the mortar (Example 9) exhibit a high dissolution rate in comparison to the capsule containing unpulverized Compound (I) (Comparative Example 1) and the dissolution property can be improved by pulverizing Compound (I) with an appropriate particle size.

### Test Example 2

To a dog under fasting, 1 capsule obtained in Example 10 and Comparative Example 2 were orally administered, respectively. Blood sampling was conducted at time intervals, and a concentration of Compound (I) in plasma was measured by high-performance liquid chromatography (HPLC). The results are shown in Fig. 2.

According to Fig. 2, it has been found that when the capsule containing Compound (I) pulverized with the jet mill (Example 10) was administered, the high concentration in plasma is provided in comparison to the administration of the capsule containing unpulverized Compound (I) (Comparative Example 2) and an absorbability in oral administration can be improved by pulverizing Compound (I) with an appropriate particle size.

Conditions for the measurement by HPLC are as follows.
Instrument for analysis: LC-10A Series (manufactured by Shimadzu Corporation)
Column: Develosil ODS-HG-5
Column temperature: room temperature
Mobile phase: 0.05 mol/L phosphate buffer (pH 3.0)/acetonitrile=3/2
Flow rate: 1 mL/minute
Detecting condition: UV 242 nm

### Test Example 3

Each of the mixtures of Compound (I) and hydroxypropylmethyl cellulose obtained in Examples 3, 6 and 7 was charged into a plastic bottle, and stored at 60°C for 10 days. A content of a decomposed product of each sample stored was measured by HPLC. The results are shown in Table 5. The average particle size of Compound (I) in the mixtures obtained in Examples 6 and 7 is estimated from the results in Example 4 in which the pulverization was conducted by the same pulverization step, respectively.

According to Table 5, it has been found that all of the samples exhibit the high stability.

**Table 5: Content of a decomposed product**

| Sample | Example 3 | Example 6 | Example 7 |
|---|---|---|---|
| Crystallinity (%) | 80 | 49 | 29 |
| Average particle size (µm) | 10 | 11 | 4 |
| Content of a decomposed product (%) | 0.0 | 0.1 | 0.1 |

### Brief Description of the Drawings

Fig. 1 shows the results of a dissolution test (puddle method) of each of the capsules obtained in Examples 8, 9 and Comparative Example 1. The ordinate shows a dissolution rate (%) of Compound (1) from the capsule, the abscissa shows a time (min), and an error bar shows a standard deviation respectively.
- □ -: showing a dissolution curve of the capsule [containing Compound (I) pulverized with the jet mill] obtained in Example 8.
- ◊ -: showing a dissolution curve of the capsule [containing Compound (I) pulverized with the mortar] obtained in Example 9.
- Δ -: showing a dissolution curve of the capsule [containing unpulverized Compound (I) ] obtained in Comparative Example 1.

Fig. 2 shows the results of the test for absorbability in oral administration to the dog regarding the capsules obtained in Example 10 and Comparative Example 2. The ordinate shows a concentration in plasma (%) of Compound (I), the abscissa shows a time (hr), and the error bar shows a standard derivation respectively.
- ○ -: showing a concentration in plasma of Compound (I) when using the capsule [containing Compound (I) pulverized with the jet mill] obtained in Example 10.
- ● -: showing a concentration in plasma of Compound (I) when using the capsule [containing unpulverized Compound (I)] obtained in Comparative Example 2.

### Industrial Applicability

The present invention provides microcrystals of Compound (I) good in, for example, solubility, absorbability and stability, a pharmaceutical formulation comprising the microcrystals.

## Claims

1. A microcrystal of (S)-(+)-3,3,3-trifluoro-2-hydroxy-2-methyl-N-(5,5,10-trioxo-4,10-dihydrothieno[3,2-c][1] benzothiepin-9-yl)propaneamide represented by the following formula 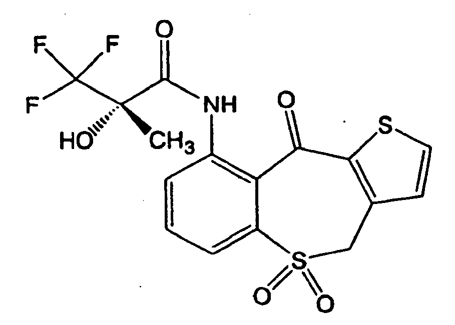 which has an average particle size of 80 µm or less.

2. The microcrystal according to Claim 1, wherein the average particle size of the microcrystal of (S)-(+)-3,3,3-trifluoro-2-hydroxy-2-methyl-N-(5,5,10-trioxo-4,10-dihydrothieno[3,2-c][1]benzothiepin-9-yl)propaneamide is from 1 to 40 µm.

3. The microcrystal of (S)-(+)-3,3,3-trifluoro-2-hydroxy-2-methyl-N-(5,5,10-trioxo-4,10-dihydrothieno[3,2-c][1] benzothiepin-9-yl)propaneamide according to Claims 1 or 2, wherein a crystallinity thereof is 15% or more.

4. The microcrystal of (S)-(+)-3,3,3-trifluoro-2-hydroxy-2-methyl-N-(5,5,10-trioxo-4,10-dihydrothieno[3,2-c][1] benzothiepin-9-yl)propaneamide according to Claims 1 or 2, wherein the crystallinity thereof is 25% or more.

5. The microcrystal of (S)-(+)-3,3,3-trifluoro-2-hydroxy-2-methyl-N-(5,5,10-trioxo-4,10-dihydrothieno[3,2-c ] [1]benzothiepin-9-yl)propaneamide according to Claims 1 or 2, wherein the crystallinity thereof is 45% or more.

6. The microcrystal of (S)-(+)-3,3,3-trifluoro-2-hydroxy-2-methyl-N-(5,5,10-trioxo-4,10-dihydrothieno[3,2-c ][1]benzothiepin-9-yl)propaneamide according to Claims 1 or 2, wherein the crystallinity thereof is 75% or more.

7. A pharmaceutical formulation comprising the microcrystals described in any one of Claims 1 to 6.

8. A process for producing the microcrystals described in any one of Claims 1 to 6, comprising a step of dry-pulverizing (S)-(+)-3,3,3-trifluoro-2-hydroxy-2-methyl -N-(5,5,10-trioxo-4,10-dihydrothieno[3,2-c][1]benzothiepin -9-yl)propaneamide which has an average particle size of 80 µm or more.

9. The process for producing the microcrystals according to the Claim 8, wherein a jet mill is used in the pulverization step.

## Patentansprüche

1. Mikrokristall von (S)-(+)-3,3,3-Trifluor-2-hydroxy-2-methyl-N-(5,5,10-trioxo-4,10-dihydrothieno[3,2-c][1]-benzothiepin-9-yl)propanamid der folgenden Formel 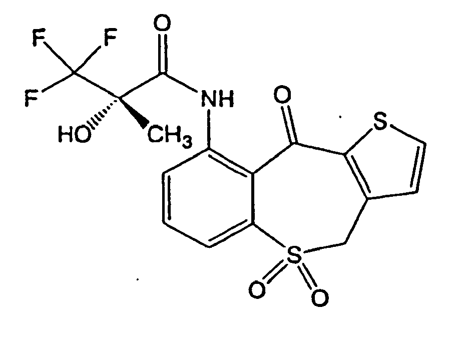 mit einer mittleren Teilchengröße von 80 µm oder weniger.

2. Mikrokristall gemäß Anspruch 1, wobei die mittlere Teilchengröße des Mikrokristalls von (S)-(+)-3,3,3-Trifluor-2-hydroxy-2-methyl-N-(5,5,10-trioxo-4,10-dihydrothieno[3,2-c][1]-benzothiepin-9-yl)propanamid von 1 bis 40 µm ist.

3. Mikrokristall von (S)-(+)-3,3,3-Trifluor-2-hydroxy-2-methyl-N-(5,5,10-trioxo-4,10-dihydrothieno[3,2-c][1]-benzothiepin-9-yl)propanamid gemäß den Ansprüchen 1 oder 2, wobei die Kristallinität davon 15% oder mehr ist.

4. Mikrokristall von (S)-(+)-3,3,3-Trifluor-2-hydroxy-2-methyl-N-(5,5,10-trioxo-4,10-dihydrothieno[3,2-c][1]-benzothiepin-9-yl)propanamid gemäß Anspruch 1 oder 2, wobei die Kristallinität davon 25% oder mehr ist.

5. Mikrokristall von (S)-(+)-3,3,3-Trifluor-2-hydroxy-2-methyl-N-(5,5,10-trioxo-4,10-dihydrothieno[3,2-c][1]-benzothiepin-9-yl)propanamid gemäß Anspruch 1 oder 2, wobei die Kristallinität davon 45% oder mehr ist.

6. Mikrokristall von (S)-(+)-3,3,3-Trifluor-2-hydroxy-2-methyl-N-(5,5,10-trioxo-4,10-dihydrothieno[3,2-c][1]-benzothiepin-9-yl)propanamid gemäß Anspruch 1 oder 2, wobei die Kristallinität davon 75% oder mehr ist.

7. Pharmazeutische Formulierung, umfassend die in einem der Ansprüche 1 bis 6 beschriebenen Mikrokristalle.

8. Verfahren zur Herstellung der in einem der Ansprüche 1 bis 6 beschriebenen Mikrokristalle, umfassend einen Schritt des Trockenpulverisierens von (S)-(+)-3,3,3-Trifluor-2-hydroxy-2-methyl-N-(5,5,10-trioxo-4,10-dihydrothieno[3,2-c][1]-benzothiepin-9-yl)propanamid, welches eine mittlere Teilchengröße von 80 µm oder mehr hat.

9. Verfahren zur Herstellung der Mikrokristalle gemäß Anspruch 8, wobei eine Jetmühle im Pulverisierungsschritt verwendet wird.

## Revendications

1. Microcristaux de (S)-(+)-3,3,3-trifluoro-2-hydroxy-2-méthyl-N-(5,5,10-trioxo-4,10-dihydro-thiéno[3,2-c][1]benzothiépin-9-yl)propanamide, représenté par la formule suivante : 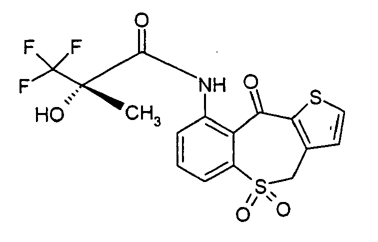 en particules dont la taille moyenne vaut au plus 80 µm.

2. Microcristaux conformes à la revendication 1, dans lesquels la taille moyenne des particules de microcristaux de (S)-(+)-3,3,3-trifluoro-2-hydroxy-2-méthyl-N-(5,5,10-trioxo-4,10-dihydro-thiéno[3,2-c][1]benzothiépin-9-yl)propanamide vaut de 1 à 40 µm.

3. Microcristaux de (S)-(+)-3,3,3-trifluoro-2-hydroxy-2-méthyl-N-(5,5,10-trioxo-4,10-dihydro-thiéno[3,2-c][1]benzothiépin-9-yl)propanamide, conformes à la revendication 1 ou 2, dont le taux de cristallinité vaut au moins 15 %.

4. Microcristaux de (S)-(+)-3,3,3-trifluoro-2-hydroxy-2-méthyl-N-(5,5,10-trioxo-4,10-dihydro-thiéno[3,2-c][1]benzothiépin-9-yl)propanamide, conformes à la revendication 1 ou 2, dont le taux de cristallinité vaut au moins 25 %.

5. Microcristaux de (S)-(+)-3,3,3-trifluoro-2-hydroxy-2-méthyl-N-(5,5,10-trioxo-4,10-dihydro-thiéno[3,2-c][1]benzothiépin-9-yl)propanamide, conformes à la revendication 1 ou 2, dont le taux de cristallinité vaut au moins 45 %.

6. Microcristaux de (S)-(+)-3,3,3-trifluoro-2-hydroxy-2-méthyl-N-(5,5,10-trioxo-4,10-dihydro-thiéno[3,2-c][1]benzothiépin-9-yl)propanamide, conformes à la revendication 1 ou 2, dont le taux de cristallinité vaut au moins 75 %.

7. Formulation pharmaceutique comprenant des microcristaux conformes à l'une des revendications 1 à 6.

8. Procédé de production des microcristaux conformes à l'une des revendications 1 à 6, qui comporte une étape de pulvérisation à sec d'un (S)-(+)-3,3,3-trifluoro-2-hydroxy-2-méthyl-N-(5,5,10-trioxo-4,10-dihydrothiéno[3,2-c][1]benzothiépin-9-yl)propanamide qui se présente en particules de taille moyenne valant au moins 80 µm.

9. Procédé de production des microcristaux, conforme à la revendication 8, dans lequel on se sert d'un broyeur à jet dans l'étape de pulvérisation.
